# EUROPEAN PATENT APPLICATION

(11) **EP 4 560 008 A1**
(43) Date of publication of application: **28.05.2025**
(21) Application number: 24853033.9
(22) Date of filing: 05.08.2024
(51) Int. Cl.: C12N 1/21, C12N 15/54, C12P 19/18, C12P 19/56, C12N 15/70, C12R 1/19

(54) **METHOD FOR EFFICIENT BIOSYNTHESIS OF REBAUDIOSIDE M2 USING GLYCOSYLTRANSFERASE**

(30) Priority: 17.08.2023 CN 202311038501
(71) Applicant: Guilin Layn Natural Ingredients Corp., Guilin, Guangxi 541199 (CN)
(72) Inventor: RAO, Yijian, Guilin, Guangxi 541199 (CN); ZHANG, Yan, Guilin, Guangxi 541199 (CN); YANG, Lifeng, Guilin, Guangxi 541199 (CN); PING, Qian, Guilin, Guangxi 541199 (CN)
(74) Representative: Sun, Yiming
(86) International application number: PCT/CN2024/109797
(87) International publication number: WO 2025/036187

(57) **Abstract**

The invention provides a method for efficient biosynthesis of rebaudioside M2 using glycosyltransferase, belonging to the technical field of biocatalytic synthesis. A glycosyltransferase UGT94D1 catalyzing rebaudioside D to synthesize rebaudioside M2 is obtained by mining, providing a new, efficient and environmentally friendly way of producing rebaudioside M2. During the whole catalytic reaction process, no by-products are produced, facilitating downstream purification and further reducing production costs.

## Description

### Technical Field

The invention relates to a method for efficient biosynthesis of rebaudioside M2 using glycosyltransferase, belonging to the technical field of biocatalytic synthesis.

### Background Art

Steviol glycoside is a diterpene glucoside extracted and purified from stevia leaves, and has the advantages of high sweetness, low calories and no side effects on the human body.

Steviol glycoside has been approved as a safe food additive by the United States, Brazil, South Korea, Japan and the European Food Safety Authority. To date, 64 steviol glycosides have been identified from stevia leaves, of which stevioside is the most abundant, accounting for 5-10% of dry weight, followed by rebaudioside A, accounting for 2-4% of dry weight. They exhibit a sweetness 250-300 times higher than sucrose, but even high-purity steviol glycosides retain their bitter properties, which significantly limits their use in the natural food additive market.

In recent years, researchers have found that the number and types of glycosyl units linked to C-13 and C-19 have significant effects on the properties of steviol glycosides. For example, rebaudioside A has an extra glucose in the position C-13, making it sweeter and more palatable than stevioside. Rebaudioside D and rebaudioside M further isolated from stevia have one and two additional glucoses linked at the position C-19, allowing them to exhibit better sweetness than rebaudioside A. Therefore, significant attention has been paid to novel steviol glycosides with different glycosyl units at these positions to find a more suitable sweetener.

UDP-glucosyltransferases (UGTs) are naturally evolved enzymes that catalyze glycosylation reactions, and transfer sugar portions from activated sugar donors to receptor molecules. UGTs have been widely used in the synthesis of steviol glycosides. In 2014, Prakash et al. reported for the first time a novel steviol glycoside derivative, rebaudioside M2, a secondary product resulting from the catalytic conversion of rebaudioside A to rebaudioside D by the glycosyltransferase UGTSL2. However, due to its low yield, mixed product composition, and high separation and purification costs, the further study of rebaudioside M2 is limited. It is therefore necessary to identify a new glycosyltransferase with high catalytic activity and regional selectivity for the catalytic synthesis of rebaudioside M2.

### Summary of the Invention

To solve the problems described above, the invention uses UGT94D1, a glucosidtransferase derived from sesame, to catalyze the synthesis of rebaudioside D to rebaudioside M2; UGT94D1 also has the activity of catalyzing the synthesis of rebaudioside D to rebaudioside M2 in the presence of uridine diphosphate glucose (UDPG), providing the possibility for further study of rebaudioside M2.

In order to solve the above technical problems, the invention adopts the following technical proposal.

The first purpose of the invention is to provide a recombinant strain, which overexpresses a sesame-derived glycosyltransferase UGT94D1.

In one embodiment, the accession number of an amino acid sequence of the glycosyltransferase is XP_011076907.1, and the accession number of a nucleotide sequence is XM_011078605.1.

In one embodiment, the recombinant strain takes either prokaryotic or eukaryotic cells as host cells.

In one embodiment, the prokaryotic host cells can be any Gram-positive or Gram-negative bacteria. Gram-positive bacteria include, but are not limited to, Bacillus, Fusiformis, Enterococcus, Geobacillus, Lactobacillus, Lactococcus, Oceanobacillus, Staphylococcus, Streptococcus and Streptomyces. Gram-negative bacteria include, but are not limited to, Campylobacter, Escherichia coli, Flavobacterium, Fusobacterium, Helicobacterium, Ilyobacter, Neisseria, Pseudomonas, Salmonella and Ureaplasma; the eukaryotic cells are fungal cells.

In one embodiment, the recombinant strain takes Escherichia coli as a host cell.

In one embodiment, the recombinant strain takes pET series as an expression vector.

In one embodiment, the recombinant strain takes pET-21b(+) as an expression vector.

The second purpose of the invention is to provide a composition, which is one or more of the glycosyltransferase UGT94D1, the recombinant strain or cell lysis buffer for the recombinant strain; the accession number of the amino acid sequence of the glycosyltransferase UGT94D1 is XP_011076907.1.

In one embodiment, the cell lysis buffer is a supernatant obtained from lytic cells after the induced expression of the recombinant strain.

The third purpose of the invention is to provide a method for catalytic synthesis of rebaudioside M2, which takes rebaudioside D as a substrate and uses the glycosyltransferase UGT94D1, the recombinant strain, and/or the composition for catalytic reaction.

In one embodiment, the method uses UDP-glucose as a glycosyl donor.

In one embodiment, a catalytic system consists of 0.1-3.0 mM of Reb D, 1-10 µM of glycosyltransferase UGT94D1, 1-10 mM of UDP-glucose, 5-20 mM of MnCl₂ and 20-80 mM of Tris.

In one embodiment, the conditions for the catalytic reaction are pH of 5.5-9.0, temperature of 30-50 degrees C, and duration of 2-10 hours.

The invention also provides an application of the glycosyltransferase UGT94D1, or the recombinant strain, or the composition, or the method in the preparation of rebaudioside M2, or products containing rebaudioside M2.

In one embodiment, the accession number of the amino acid sequence of the glycosyltransferase UGT94D1 is XP_011076907.1, and the accession number of the nucleotide sequence is XM_011078605.1.

**Beneficial effects:** the invention uses the nucleic acid sequence for encoding the glycosyltransferase UGT94D1 to prepare recombinant proteins that can catalyze rebaudioside D to generate rebaudioside M2; the recombinant proteins prepared can take UDPG as a glycosyl donor, after which rebaudioside D is taken as the substrate for the glycosylated synthesis of rebaudioside M2, and no byproducts are produced in the catalytic reaction process, facilitating downstream purification and reducing production costs. The yield and production of rebaudioside M2 are 90% and 2.32 g/L.

### Brief Description of the Drawings

Fig. 1 shows the catalytic biosynthetic route of rebaudioside D to rebaudioside M2 by the glycosyltransferase UGT94D1.
Fig. 2 shows the protein expression and purification analysis of the glycosyltransferase UGT94D1 in Embodiment 2. Lane 1: Marker; Lane 2: non-IPTG induced expression sample; Lane 3: crude enzyme; Lane 4: crude enzyme supernatant; Lane 5: crude enzyme precipitation; Lane 6: purification of penetrating fluid; Lane 7: washing of impure protein sample; Lane 8: objective protein elution sample.
Fig. 3 is the UPLC analysis chart for the catalytic synthesis of rebaudioside D to rebaudioside M2 by the glycosyltransferase UGT94D1 in Embodiment 3.
Fig. 4 shows the mass spectrometry of the rebaudioside D glycosylation product rebaudioside M2 in Embodiment 3.
Fig. 5 shows the H NMR spectroscopy of the product rebaudioside M2 in Embodiment 4.
Fig. 6 shows the C NMR spectroscopy of the product rebaudioside M2 in Embodiment 4.
Fig. 7 shows the COSY NMR spectroscopy of the product rebaudioside M2 in Embodiment 4.
Fig. 8 shows the TOCSY NMR spectroscopy of the product rebaudioside M2 in Embodiment 4.
Fig. 9 shows the HSQC NMR spectroscopy of the product rebaudioside M2 in Embodiment 4.
Fig. 10 shows the HMBC NMR spectroscopy of the product rebaudioside M2 in Embodiment 4.
Fig. 11 shows the ROESY NMR spectroscopy of the product rebaudioside M2 in Embodiment 4.

### Detailed Description of the Invention

The invention is further described below in combination with the drawings to the specification and the embodiments, but the embodiments are not intended to limit the invention in any form. Unless otherwise specified, reagents, methods and equipment used in the invention are conventional reagents, methods and equipment commonly used in the technical field.

Unless otherwise specified, reagents and materials used in the following embodiments are commercially available or can be prepared by known methods.

### Media involved in the following embodiments:

LB solid media: 10 g/L peptone, 5 g/L yeast powder, 10 g/L NaCl, 20 g/L agar powder. 2 × YT liquid medium: 16 g/L peptone, 10 g/L yeast powder, 5 g/L NaCl.

### Methods involved in the following embodiments:

Determination of enzymatic properties of glycosyltransferase: the kinetic analysis of rebaudioside D by glycosyltransferase UGT94D1 was carried out in a 200 µL reaction system, which contained 5 mM of UDPG, 10 mM of MnCl₂, 50 mM of pH 8.0 Tris-HCI and 5 µg of purified protein sample (glycosyltransferase UGT94D1), and the concentration of rebaudioside D was 0-5 mM. The reaction temperature was 35 degrees C and the reaction time was 2 h; then heat at 95 degrees C for 5 minutes and quench the reaction, and dilute the reaction mixture with 2-fold methanol; centrifuge at 20000×g for 5 minutes to remove precipitate; filter the supernatant by 0.22 µm filter membrane for UPLC analysis.

Definition of enzyme activity: the volume of enzyme required to synthesize 1 µM of rebaudioside M2 within 1 hour.

Determination of yield of rebaudioside M2: prepare a standard solution of rebaudioside M2 with different concentrations (0 mM, 0.05 mM, 0.1 mM, 0.15 mM, 0.2 mM, 0.25 mM, 0.3 mM), analyze the standard solution by UPLC, and obtain the standard concentration curve equation for rebaudioside M2: y=2383564.28571x+1556.96429, and R²=0.99664. Obtain the yield of rebaudioside M2 according to the standard curve. Yield = Actual yield of rebaudioside M2 / theoretical yield of rebaudioside M2.

Waters Acquity UPLC system: BEH C18 1.7 µM chromatographic column (2.1x50 mm) was used; the liquid phase conditions were as follows: organic phase: acetonitrile; aqueous phase: ultrapure water; flow rate: 0.3 mL/min; column temperature: 40 degrees C; UV detection wavelength: 210 nm; detection procedure: 0-1 minute for 15% organic phase, 6 minutes for 40% organic phase, 7-8 minutes for 15% organic phase.

### Embodiment 1: gene acquisition of glycosyltransferase UGT94D1 and creation of recombinant strain

The amino acid sequence (accession number: XP_011076907.1) and nucleotide sequence (accession entry number: XM_011078605.1) of Bacillus glycosyltransferase were downloaded from Genbank; Yixin Biotechnology Co., Ltd. carried out gene synthesis and linked to the polyclonal enzyme cleavage site of the vector pET-21b(+) to obtain the recombinant plasmid pET-21b(+)-UGT94D1.

Sequence and convert the obtained plasmid pET-21b(+)-UGT94D1 into competent cells of E. coli BL21(DE3), then use LB solid plate containing 100 µg/mL ampicillin for screening to obtain the recombination strain E.coli BL2I (DE3)pET-21b(+)-UGT94D1.

### Embodiment 2: induced expression of recombinant strain and purification of objective proteins

Inoculate the recombinant strain E. coli BL2I (DE3) pET-21b(+)-UGT94D1 created in Embodiment 1 into 1 L of 2 ×YT liquid medium containing 100 µg/mL ampicillin, and culture at 135 rpm and 37 degrees C until OD₆₀₀ is 0.6-0.8, lower the culture temperature to 18 degrees C, add isopropyl-β-thiogalactopyranoside (IPTG) with a final concentration of 0.1 mmol/L, and perform an induced culture for 8 hours.

Centrifuge the induced expression bacteria solution (7000 rpm, 7 minutes, 4 degrees C), discard the supernatant, and collect bacterial cells. Resuspend the bacterial cells using 10 mL of lysis buffer (50 mmol/L pH 8.0 Tris-HCI, 300 mmol/L NaCl, 10 mmol/L imidazole, 10% glycerol) per 1 g of bacterial cells. Use a high-pressure homogenizer for crushing, then centrifuge the crushed bacteria solution (40000×g, 30 minutes), and take the supernatant to obtain the crude enzyme.

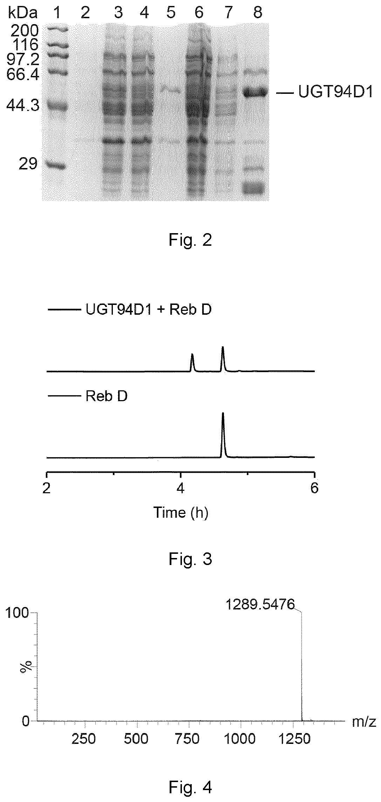

Use an Ni+ column to purify the crude enzyme by affinity chromatography, wash the impure protein with 10-fold lysis buffer after sample loading, and elute objective proteins using elution buffer (50 mmol/L pH 8.0 Tris-HCI, 300 mmol/L NaCl, 250 mmol/L imidazole, 10% glycerin). Collect the eluted objective proteins, desalt with desalting buffer (25 mmol/L Tris-HCI, 150 mmol/L NaCl, 10% glycerol) using a HistrpTM 5 mL desalting column, and then concentrate to 10 mg/mL for subsequent reaction. Detect the purified proteins by means of 10% SDS-PAGE gel electrophoresis. The results are shown in Fig. 2; a pure enzyme with clear objective bands and accurate protein size was successfully obtained; the Kₘ value and k_{cat} value of UGT94D1 for rebaudioside D were respectively 0.89± 0.05mM and 0.33±0.08 min⁻¹, while the enzyme activity of pure enzyme UGT94D1 was 2.12 U/mg.

### Embodiment 3: glycosylation of UGT94D1 for catalytic synthesis of rebaudioside D to rebaudioside M2

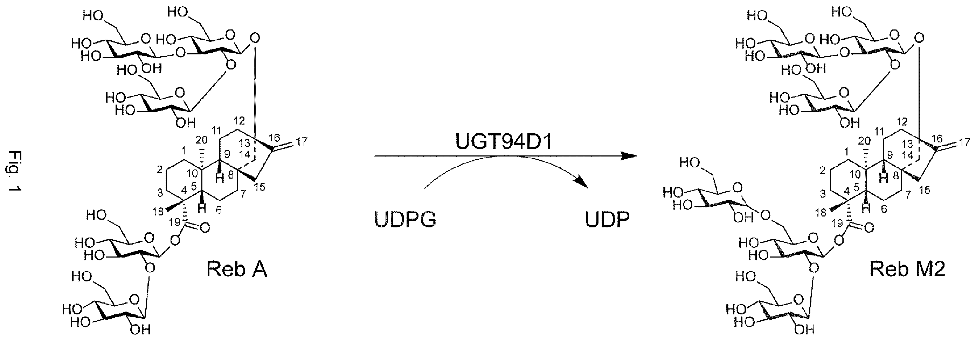

Use the purified glycosyltransferase UGT94D1 obtained in Embodiment 2 for glycosylation (Fig. 1).

Carry out glycosylation in a 200 µL reaction system, which consists of 50 mmol/L pH 8.0 Tris, 5 mmol/L UDPG, 10 mmol/L MnCl₂ and 2 mmol/L rebaudioside D; the concentration of the pure enzyme UGT94D1 obtained in Embodiment 2 was 5 µM; react at 35 degrees C for 4 hours.

Then heat at 95 degrees C for 5 minutes and quench the reaction; dilute the reaction mixture with 2-fold methanol; centrifuge at 20000×g for 5 minutes to remove precipitate; filter the supernatant using a 0.22 µm filter membrane for UPLC and LC-MS analysis. Waters Acquity UPLC system used BEH C18 1.7 µM chromatographic column (2.1×50 mm), with the liquid phase conditions as follows: organic phase: acetonitrile; aqueous phase: ultrapure water; flow rate: 0.3 mL/min; column temperature: 40 degrees C; UV detection wavelength: 210 nm; detection procedure: 0-1 minute for 15% organic phase, 6 minutes for 40% organic phase, 7-8 minutes for 15% organic phase.

Results can be seen from the liquid phase analysis, as shown in Fig. 3. Compared with the rebaudioside D standard, it can be seen that new products were obviously produced in the reaction system, with no by-products. The reaction mixture was analyzed by mass spectrometry (MS) (Fig. 4), and the results in the negative ion mode of LC-MS showed that a [M-H]⁻ ion peak existed at m/z 1289.5421, with the corresponding molecular formula of C₅₆H₉₀O₃₃, indicating that the product was rebaudioside M2, a monosaccharide derivative of rebaudioside D. By means of HPLC quantitative analysis, the yield and production of rebaudioside M2 were found to be 90% and 2.32 g/L.

### Embodiment 4: Structural identification of novel monosaccharide derivatives for rebaudioside D

New derivatives were prepared with glycosyltransferase UGT94D1 by large-scale (100 mL) glycosylation. The reaction system was as follows: 2 mM of Reb D, 10 µM of glycosyltransferase, 5 mM of UDPG, 10 mM of MnCl₂ and 50 mM of pH8.0 Tris. Make the reaction mixture reacted at 35 degrees C for 24 hours, then heat at 95 degrees C for 5 minutes and quench the reaction, and centrifuge at 20000×g for 5 minutes to remove precipitate; filter the supernatant using a 0.22 µm filter membrane, and purify by a semi-preparation high performance liquid chromatography (HPLC) system, which used Shim-pack GIST C18 chromatographic column (10×250 mm, 5µm, SHIMADZU, Japan); the liquid phase conditions were as follows: organic phase: acetonitrile; aqueous phase: ultrapure water; flow rate: 5 mL/min; time procedure: 0-28 minutes for 23% organic phase, 28.5-30.5 minutes for 60% organic phase, 31-35 minutes for 23% organic phase; column temperature: 40 degrees C; UV detection wavelength: 210 nm. The sample obtained was dissolved in heavy water, and the overall structure of the products was analyzed by 1D (¹H and ¹³C) and 2D NMR (COSY, TCOSY, HSQC, HMBC and ROESY) spectra. Data were collected using a Bruker Avance III 600 MHz spectrometer (Bruker BioSpin, Karlsruhe, Germany), with a ¹H spectrum detection frequency of 600 MHz and ¹³C spectrum detection frequency of 151 MHz.

The chemical shifts of H and C for new derivatives were assigned by 1D and 2D HMR in detail, as shown in Table 1. The structural formula of the product resulting from catalytic synthesis of Reb D by UGT94D1 is 13-[(2-O-β-D-glucopyranosyl-3-O-β-D-glucopyranosyl-β-D-glucopyranosyl)oxy] ent-kaur-16-en-19-oic acid-[(2-O-β-D-glucopyranosyl-6-O-β-D-glucopyranosyl-β-D-glucopyranosyl) ester].

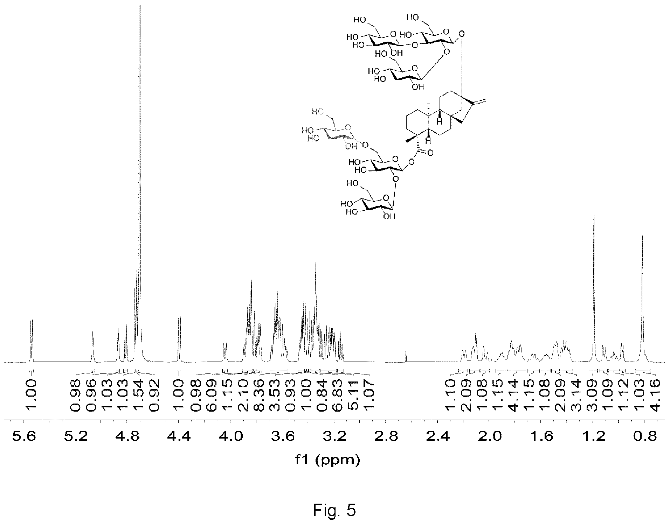

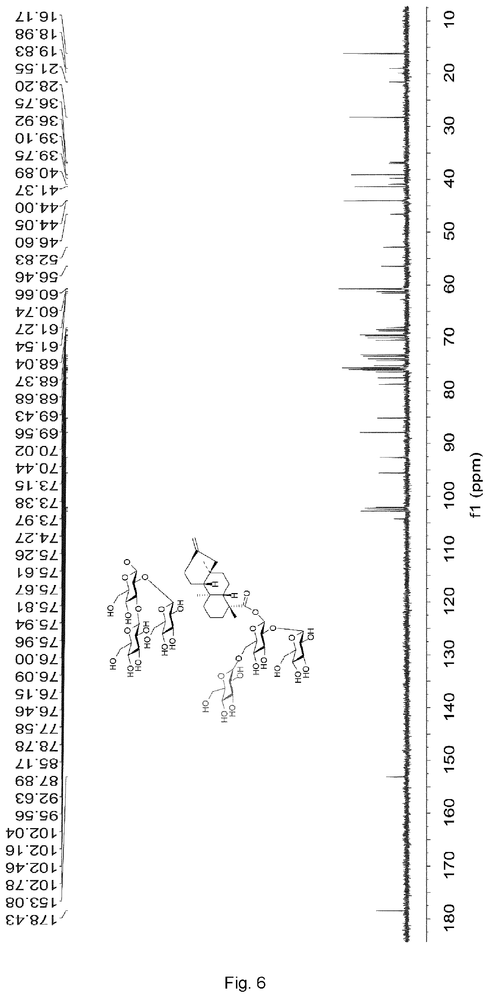

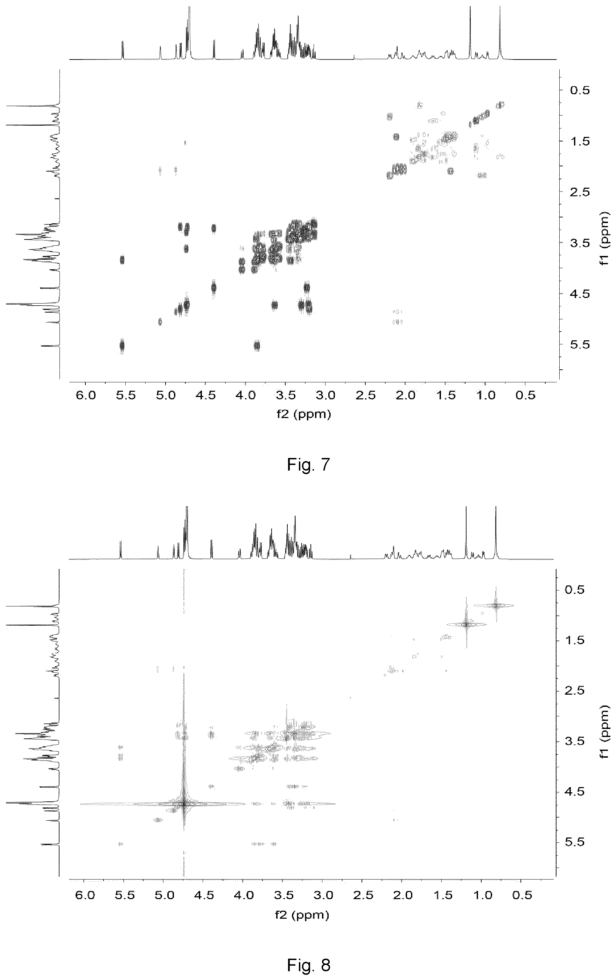

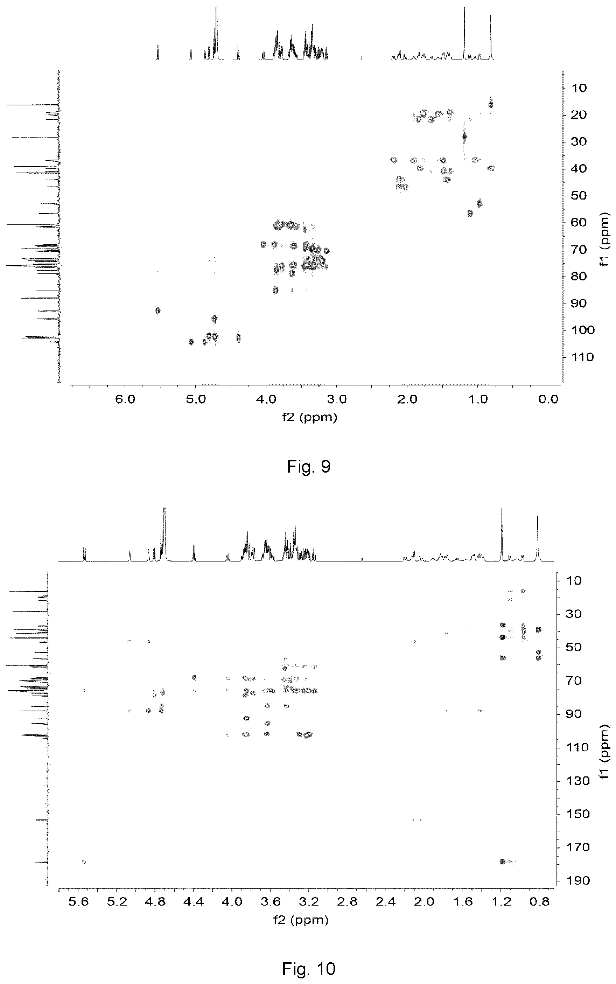

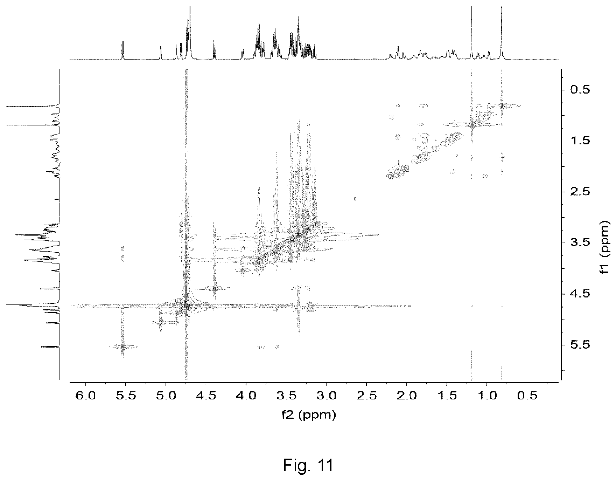

The structure of this product was consistent with that of Reb M2 as reported in the literature, and the resulting product was consistent with rebaudioside M2 as reported in the literature (Fig. 5 to Fig. 11).

**Table 1 ¹H and ¹³C Chemical Shift Assignments (D2O)**

| Position | δH (*J*) | δC | Position | δH (*J*) | δC |
|---|---|---|---|---|---|
| 1 | 39.10 | 0.81 (m) | II-1 | 95.56 | 4.74 (d, 7.6) |
| 2 | | 1.81 (m) | II-2 | 78.78 | 3.62 (m) |
| | 18.98 | 1.37 (m) | II-3 | 85.17 | 3.87 (m) |
| | | 1.77 (m) | II-4 | 68.37 | 3.43 (m) |
| 3 | 36.75 | 1.03 (m) | II-5 | 77.58 | 3.84 (m) |
| | | 2.18 (d, 13.2) | II-6 | 60.66 | 3.32 (m) |
| 4 | 40.98 | | II-6 | | 3.65 (m) |
| 5 | 56.46 | 1.11 (d, 12.4) | III-1 | 102.04 | 4.81 (d, 7.9) |
| 6 | 21.55 | 1.62 (m) | III-2 | 73.38 | 3.20 (m) |
| 7 | | 1.83 (m) | III-3 | 75.81 | 3.40 (m) |
| | 41.37 | 1.38 (m) | III-4 | 70.44 | 3.14 (m) |
| | | 1.44 (m) | III-5 | 75.94 | 3.31 (m) |
| 8 | 44.00 | | III-6 | 61.27 | 3.62 (m) |
| 9 | 52.83 | 0.97 (d, 8.0) | | | 3.81 (m) |
| 10 | 39.75 | | IV-1 | 102.16 | 4.73 (d, 7.8) |
| 11 | 19.83 | 1.52 (m) | IV-2 | 73.15 | 3.28 (m) |
| 12 | | 1.57 (m) | IV-3 | 75.96 | 3.35 (m) |
| | 36.92 | 1.48 (m) | IV-4 | 69.56 | 3.32 (m) |
| | | 1.89 (m) | IV-5 | 78.78 | 3.63 (m) |
| 13 | 87.89 | | IV-6 | 61.54 | 3.44 (m) |
| 14 | 44.05 | 1.41 (m) | | | 3.67 (m) |
| 15 | | 2.12 (m) | V-1 | 102.46 | 4.72 (d, 7.9) |
| | | 2.03 (d, 17.4) | V-2 | 75.26 | 3.21 (m) |
| 16 | | 2.10 (m) | V-3 | 75.67 | 3.41 (m) |
| | 153.08 | | V-4 | 70.02 | 3.25 (m) |
| 17 | 104.27 | 4.86 (s) | V-5 | 76.00 | 3.45 (m) |
| | | 5.06 (s) | V-6 | 60.66 | 3.64 (m) |
| 18 | 28.20 | 1.18 (3H, s) | | | 3.86 (m) |
| 19 | 178.43 | | VI-1 | 102.78 | 4.39 (d, 7.9) |
| 20 | 16.17 | 0.81 (3H, s) | VI-2 | 74.27 | 3.24 (m) |
| I-1 | 92.63 | 5.53 (d, 7.9) | VI-3 | 75.61 | 3.38 (m) |
| I-2 | 77.58 | 3.83 (m) | VI-4 | 69.56 | 3.34 (m) |
| I-3 | 76.15 | 3.76 (m) | VI-5 | 76.09 | 3.37 (m) |
| I-4 | 68.68 | 3.58 (m) | VI-6 | 60.74 | 3.79 (m) |
| I-5 | 76.46 | 3.60 (m) | | | 3.83 (m) |
| I-6 | 68.04 | 3.89 (m) | | | |
| | | 4.03 (m) | | | |

Although the invention has disclosed the preferred embodiments, they are not intended to limit the invention. Any person familiar with the technology can make various changes and modifications within the spirit and scope of the invention, so the scope of protection of the invention shall be considered as defined in the claims.

## Claims

1. A recombinant strain, **characterized in that** an expression is derived from a glycosyltransferase UGT94D1 of sesame, and the NCBI accession number of an amino acid sequence of the glycosyltransferase UGT94D1 is XP_011076901.7.

2. The recombinant strain according to claim 1, **characterized in that** the recombinant strain takes Escherichia coli as a host cell.

3. The recombinant strain according to claim 1, **characterized in that** the recombinant strain takes pET series as an expression vector.

4. The recombinant strain according to claim 3, **characterized in that** the recombinant strain takes pET-21b(+) as an expression vector.

5. A composition, **characterized in that** the composition is one or more of the glycosyltransferase UGT94D1, the recombinant strain in any of claims 1-4 or cell lysis buffer for the recombinant strain in any of claims 1-4; the accession number of the amino acid sequence of the glycosyltransferase UGT94D1 is XP_011076907.1.

6. The composition according to claim 5, **characterized in that** the cell lysis buffer is a supernatant obtained from lytic cells after the induced expression of the recombinant strain in any of claims 1-4.

7. A method for catalytic synthesis of rebaudioside M2, **characterized in that** rebaudioside D is taken as a substrate, and the glycosyltransferase UGT94D1, the recombinant strain in any of claims 1-4, and/or the composition in claim 5 or 6 are used for catalytic reaction; the accession number of the amino acid sequence of the glycosyltransferase UGT94D1 is XP_011076907.1.

8. The method according to claim 7, **characterized in that** UDP-glucose is taken as a glycosyl donor.

9. The method according to claim 8, **characterized in that** a catalytic system consists of 0.1-3.0 mM of Reb D, 1-10 µM of glycosyltransferase UGT94D1, 1-10 mM of UDP-glucose, 5-20 mM of MnCl₂ and 20-80 mM of Tris.

10. Application of the glycosyltransferase UGT94D1, the recombinant strain in any of claims 1-4, or the composition in claim 5 or 6, or the method in any of claims 7-9 in the preparation of rebaudioside M2 or products containing rebaudioside M2; the accession number of the amino acid sequence of the glycosyltransferase UGT94D1 is XP_011076907.1.
